# EUROPÄISCHE PATENTSCHRIFT

(11) **EP 1 804 923 B1**
(45) Veröffentlichungstag und Bekanntmachung des Hinweises auf die Patenterteilung: **01.04.2009**
(21) Anmeldenummer: 05806494.0
(22) Anmeldetag: 24.10.2005
(51) Int. Cl.: A61Q 19/00, A61K 8/97, A61K 8/98

(54) **FEUCHTIGKEITSREGULIERENDES KOSMETIKUM**
MOISTURE-REGULATING COSMETIC
COMPOSITION COSMETIQUE REGULATRICE D'HUMIDITE

(30) Priorität: 25.10.2004 DE 102004052404
(43) Veröffentlichungstag der Anmeldung: 11.07.2007
(73) Patentinhaber: Coty Prestige Lancaster Group GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Ziebig, Marlene
(86) Internationale Anmeldenummer: PCT/EP2005/011621
(87) Internationale Veröffentlichungsnummer: WO 2006/045623

(56) Entgegenhaltungen:
- EP-B- 1 185 244

## Beschreibung

Die Erfindung betrifft eine kosmetische Zusammensetzung mit feuchtigkeitsregulierender Wirkung.

Aus der EP 1185244 ist ein enzymhaltiges Kosmetikum mit regenerativer Wirkung für die Haut bekannt, das pflanzliche Milchwässer von Bananen, Löwenzahn, Windengewächsen, Mohn und/oder Soja enthält zusammen mit einem Kokosmilchkonzentrat und einem alkoholischen Extrakt von Honig/Reiskeimöl/Reisschalenöl. Neben der zellregenerierenden Wirkung ist auch eine feuchtigkeitsregulierende Wirkung beschrieben, die für acht Stunden im wesentlichen anhält.

Für trockene Haut stehen bei kosmetischen Zusammensetzungen die feuchtigkeitsregulierenden Wirkungen meist im Vordergrund, so dass das Bedürfnis besteht, nicht nur höhere sondern auch deutlich länger anhaltende Feuchthaltewirkungen zu erreichen.

Aufgabe der Erfindung ist es, eine kosmetische Zusammensetzung auf Basis pflanzlicher Wirkbestandteile zu entwickeln, die eine langanhaltende Feuchthaltewirkung von bis zu 24 Stunden hat.

Erfindungsgemäß umfasst das Kosmetikum
(a) eine wässrige Pflanzenmilch einer Frucht von Elaeis guinensis,
(b) eine wässrige Pflanzenmilch der Blätter von Phoenix canariensis,
(c) eine wässrige Pflanzenmilch von Reisschalen von Oryza sativa,
(d) eine wässrige Pflanzenmilch der Frucht von Cocos nucifera,
(e) einen Honigkomplex, bestehend aus Akazienhonig, Eukalyptushonig, Pinienhonig und Lavendelhonig, sowie einen restlichen Anteil bis zu 100 Gew-% kosmetische Hilfsstoffe, Trägerstoffe und weitere Wirkstoffe.

Es wurde gefunden, dass die durch kaltes Pressen erhaltene Palmfruchtmilch von Elaeis guinensis, einer westafrikanischen Palme, eine Erhöhung des Corneozytenvolumens und eine Verbesserung des transepidermalen Wasserverlustes (TEWL) im Zusammenwirken mit den anderen Bestandteilen bewirken kann. Der Anteil der Palmfruchtmilch liegt im Bereich von 0,1 bis 10 Gew-%, bezogen auf das Gesamtgewicht des Kosmetikums, vorzugsweise im Bereich von 0,5 bis 5 Gew-%. Besonders bevorzugt sind Gehalte von 0,5 bis 2,8 Gew-%.

Eine weitere Verbesserung der Feuchthaltewirkung im Zusammenwirken mit den anderen Bestandteilen des Kosmetikums wurde durch die wässrige Palmblattmilch von Phoenix canariensis gefunden. Der Anteil der Palmblattmilch liegt im Bereich von 0,1 bis 5,0 Gew-%, bezogen auf das Gesamtgewicht des Kosmetikums, vorzugsweise im Bereich von 0,6 bis 2,0 Gew-%.

Reisschalenmilch von Oryza sativa, die auch Phytosterole und Proteine enthält und antioxidativ sowie feuchthalteverstärkend wirkt, wird mit einem Anteil im Bereich von 0,1 bis 5,0 Gew-%, bezogen auf das Gesamtgewicht des Kosmetikums hinzugesetzt, vorzugsweise im Bereich von 0,5 bis 2,5 Gew-%.

Zum Erhalt der zellregenerierenden Wirkung ist die bereits bekannte wässrige Pflanzenmilch der Frucht von Cocos nucifera enthalten, die mittels der darin enthaltenen Enzyme zum Abbau von Fettsäuretriglyceriden beiträgt. Deren Anteil liegt im Bereich von 0,1 bis 7,0 Gew-%, bezogen auf das Gesamtgewicht des Kosmetikums, vorzugsweise im Bereich von 0,2 bis 3,0 Gew-%.

Besonders vorteilhaft für die langanhaltende Wirkung bei der Feuchtigkeitsregulierung ist der Einsatz eines Honigkomplexes aus Akazienhonig, Eukalyptushonig, Pinienhonig und Lavendelhonig, wobei der Anteil der Honigkomponenten untereinander im Verhältnis 1:0,5-1,4:0,4-1,3:0,6-1,8 liegen kann, und der Anteil des Komplexes in dem Kosmetikum im Bereich von 0,1 bis 5,0 Gew-%, bezogen auf das Gesamtgewicht des Kosmetikums, vorzugsweise im Bereich von 0,1 bis 2,5 Gew-% liegt.

In klinischen Studien wurde gefunden, dass die Hauthydratation bei einmaliger Anwendung und einer unmittelbar danach gemessenen 100%igen Erhöhung gegenüber dem Ausgangswert nur sehr langsam abfällt, nach 8 Stunden noch immer mit 35 % über dem Ausgangswert liegt und nach 24 noch mit 15-18 % über dem Ausgangswert eine deutliche Verbesserung zeigt. Auch die Ergebnisse der Studie bei dauernder täglicher Anwendung über einen Zeitraum von 4 Wochen lässt erkennen, dass der Wassergehalt der Haut im Mittel um 40 % höher liegt als die Ausgangswerte und selbst bei Absetzen der täglichen Behandlung nur sehr langsam zurückgeht.

Das erfindungsgemäße Kosmetikum kann in bestimmten kosmetischen Anwendungsformen eingesetzt werden. Derartige Anwendungsformen sind z.B. Lotion, Gel, Tagescreme, Nachtcreme, Sonnencreme, Sonnenmilch, After-sun Produkt, Spray, Balm, Maske, Makeup, Handcreme, Körperpflegeprodukt und /oder Haarprodukt.

Die entsprechende Anwendungsform enthält neben den genannten Wirkprodukten kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Es können auch weitere zusätzliche Wirkstoffe neben den genannten Wirkprodukten eingesetzt werden. Zu diesen zusätzlichen Wirkstoffen gehören z.B. anorganische und organische Lichtschutzmittel, Radikalfänger, Feuchthaltemittel, Vitamine, Enzyme, weitere pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109, Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Superoxiddismutase; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure.

Es ist weiterhin vorteilhaft, den kosmetischen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bos-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂ SiO₂, ZnO, Fe₂O₃, ZrO₂, MnO, Al₂O₃, die auch im Gemisch verwendet werden können.

Besonders bevorzugt als anorganische Pigmente sind agglomerierte Substrate von TiO₂ und/oder ZnO, die einen Gehalt an sphärischen und porösen SiO₂-Teilche aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilche mit den anderen anorganischen Stoffen definierte Agglomerate mit einer Teilchengröße im Bereich von 0,06 µm bis 5 µm bilden (gemäß WO99/06012).

Ein weiterer bevorzugter Wirkstoffzusatz für das erfindungsgemäße Kosmetikum ist eine Zubereitung mit hohem Radikalschutzfaktor (siehe WO99/66881) mit einem Gehalt an einem durch Extraktion der Rinde von Quebracho blanco und nachfolgender enzymatischer Hydrolyse gewonnenem Produkt (Radicalys^{®} von Greentech, Frankreich), und einem nichtionischen, kationischen oder anionischen Hydro-Gel oder Gemisch von Hydro-Gelen, und einem oder mehreren Phospholipiden, und Wasser. Diese Zubereitung kann gegebenenfalls noch ergänzt sein durch ein Superoxiddismutase-haltiges Hefeaufschlußprodukt und/oder Cyclodextrine (WO 94/13783).

Die für die Erfindung eingesetzten Öle können übliche kosmetische Öle sein, wie ein Mineralöl; hydriertes Polyisobuten, synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianußöl, Rizinusöl, Kakaobutter, Kokosnußoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflußt, wie Transparenzgrad, Weichheit, Härte, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate, Propylene glycol dicaprylate 2,30 dicaprate, Tridecyl stearate/neopentyl glycol dicaprylate dicaprate/tridecyl trimellitate, Neopentyl glycol dioctanoate, Isopropyl myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure etc.

Als Feuchthaltemittel sind bevorzugt Glycerin, Butylenglycol, Propylenglycol und Gemische davon.

Für die Emulsionsbildung können als oberflächenaktive Mittel anionische, amphotere, nichtionische oder kationische oberflächenaktive Mittel oder Gemische davon eingesetzt werden.

Die Erfindung betrifft auch die Verwendung eines Kosmetikums zur langanhaltenden Feuchthaltung der Haut, wobei das Kosmetikum eine wässrige Pflanzenmilch der Frucht von Elaeis guinensis, eine wässrige Pflanzenmilch der Blätter von Phoenix canariensis, eine wässrige Pflanzenmilch von Reisschalen von Oryza sativa, eine wässrige Pflanzenmilch der Frucht von Cocos nucifera und einen Honigkomplex, bestehend aus Akazienhonig, Eukalyptushonig, Pinienhonig und Lavendelhonig, umfasst.

Die Erfindung soll nachstehend durch Beispiele näher erläutert werden. Alle Angaben erfolgen in Gewichtsprozent, sofern nichts anderes angegeben ist.

### Beispiel 1 Creme

| **Phase A** | |
|---|---|
| Wasser | q.s. ad 100 |
| Glycerin | 2,5 |

| **Phase B** | |
|---|---|
| Beheneth-25 | 3,0 |
| Cetearyl Alcohol | 2,0 |
| Shea Butter | 9,0 |

| **Phase C** | |
|---|---|
| Honigkomplex* | 1,0 |
| Palmfruchtmilch Elaeis guinensis, | 0,4 |
| Palmblattmilch Phoenix canariensis | 0,6 |
| Reisschalenmilch Oryza sativa | 0,5 |
| Palmfruchtmilch Cocos nucifera | 0,5 |
| Parfüm | 0,5 |
| Konservierungsmittel | 0,5 |

| | |
|---|---|
| *Akazienhonig, Eukalyptushonig, Pinienhonig und Lavendelhonig im Verhältnis 1:1:1,1:0,7 Die Phasen A und B werden jeweils separat bei etwa 70 °C durch Mischen hergestellt und für etwa 20 Minuten homogenisiert. Nach Abkühlen auf 35 °C wird die Phase C unter Rühren hinzugegeben. | |

### Beispiel 2 Lotion

| **Phase A** | |
|---|---|
| Wasser | q.s. ad 100 |
| Glycerin | 0,5 |
| Xanthan Gum | 0,5 |

| **Phase B** | |
|---|---|
| Cyclopentasiloxane | 5,0 |
| Dicaprylyl Carbonate | 5,0 |

| **Phase C** | |
|---|---|
| Honigkomplex* | 0,9 |
| Palmfruchtmilch Elaeis guinensis, | 1,0 |
| Palmblattmilch Phoenix canariensis | 2,5 |
| Reisschalenmilch Oryza sativa | 3,0 |
| Palmfruchtmilch Cocos nucifera | 0,8 |
| Parfüm | 0,5 |
| Konservierungsmittel | 0,5 |

| | |
|---|---|
| * Akazienhonig, Eukalyptushonig, Pinienhonig und Lavendelhonig im Verhältnis 1:1,2:0,9:0,7 Die Herstellung der Lotion erfolgte wie im Beispiel 1. | |

### Beispiel 3 Nachtcreme

| **Phase A** | |
|---|---|
| PEG 20 Methyl Glucose Sesquisstearate | |
| 99,97 %/Tocopherol 0,023 % | 5,0 |
| Petrolatum | 2,0 |
| Shea Butter | 1,0 |

| **Phase B** | |
|---|---|
| Wasser | q.s. ad 100 |
| Glycerin | 5,0 |

| **Phase C** | |
|---|---|
| Silicone | 6,0 |

| **Phase D** | |
|---|---|
| Honigkomplex* | 3,0 |
| Palmfruchtmilch Elaeis guinensis | 1,0 |
| Palmblattmilch Phoenix canariensis | 0,5 |
| Reisschalenmilch Oryza sativa | 1,0 |
| Palmfruchtmilch Cocos nucifera | 1,8 |
| Parfüm | 0,5 |
| Konservierungsmittel | 0,5 |

| | |
|---|---|
| * Akazienhonig, Eukalyptushonig, Pinienhonig und Lavendelhonig im Verhältnis 1:1:0,8:0,8. Die Phasen A und B werden jeweils separat bei etwa 70 °C durch Mischen hergestellt und für etwa 20 Minuten homogenisiert. Nach Abkühlen auf 50 °C wird die Phase C unter Rühren hinzugegeben. | |

Danach wird zu den Gemisch bei etwa 35 °C die Phase D unter Rühren zugegeben.

### Beispiel 4 Vergleichsversuche

Es wurden Haut-Feuchtigkeitsmessungen mit einem Corneometer bei 20 Probandinnen mit trockener Mischhaut durchgeführt. Eingesetzt wurde ein Corneometer CM 825 (Courage&Khazaka, Deutschland) bei 21 °C und 52 % relativer Luftfeuchtigkeit. 2 Stunden nach einer vorherigen Hautreinigung wurden die unterschiedlichen Cremes aufgetragen. Die Ergebnisse der Feuchthaltung in % sind als Mittelwerte in der folgenden Tabelle aufgeführt.

**Tabelle 1**

| Zeit | Creme von Bsp. 1 ohne Honigkomplex | Creme von Bsp. 1 mit Honigkomplex | Creme von Bsp. 1 EP 1185244 |
|---|---|---|---|
| 0,5 h | 35 % | 63% | 94% |
| 2 h | 26 % | 50% | 90% |
| 8 h | 15 % | 31 % | 88 % |
| 24 h | 12 % | 19 % | 2 % |

Der Vergleich zeigt eine deutlich bessere Feuchtigkeitshaltung der Creme von Beispiel 1 der vorliegenden Erfindung mit dem Honigkomplex gegenüber einer Creme ohne diesen Komplex. Die Creme der EP 1185244 zeigte zwar nach 8 Stunden einen höheren Feuchtigkeitswert als die der vorliegenden Erfindung, fiel jedoch nach 24 h auf nahe null ab, währen die erfindungsgemäße Creme noch immer Feuchtigkeitswerte von 20 % hatte.

## Patentansprüche

1. Feuchtigkeitsregulierendes Kosmetikum, **dadurch gekennzeichnet, dass** es umfasst
(a) eine wässrige Pflanzenmilch der Frucht von Elaeis guinensis,
(b) eine wässrige Pflanzenmilch der Blätter von Phoenix canariensis,
(c) eine wässrige Pflanzenmilch von Reisschalen von Oryza sativa,
(d) eine wässrige Pflanzenmilch der Frucht von Cocos nucifera,
(e) einen Honigkomplex, bestehend aus Akazienhonig, Eukalyptushonig, Pinienhonig und Lavendelhonig,
und den Rest zu 100 Gew-% kosmetische Hilfsstoffe, Trägerstoffe und weitere Wirkstoffe, wobei die Prozentangaben auf das Gesamtgewicht des Kosmetikums bezogen sind.

2. Kosmetikum nach Anspruch 1, **dadurch gekennzeichnet, dass** der Anteil der Pflanzenmilch der Frucht von Elaeis guinensis im Bereich von 0,1 bis 10 Gew-%, bezogen auf das Gesamtgewicht des Kosmetikums, vorzugsweise im Bereich von 0,5 bis 5 Gew-% liegt.

3. Kosmetikum nach Anspruch 1, **dadurch gekennzeichnet, dass** der Anteil der Palmblattmilch von Phoenix canariensis im Bereich von 0,1 bis 5 Gew-%, bezogen auf das Gesamtgewicht des Kosmetikums, vorzugsweise im Bereich von 0,6 bis 2 Gew-% liegt.

4. Kosmetikum nach Anspruch 1, **dadurch gekennzeichnet, dass** der Anteil der Reisschalenmilch von Oryza sativa im Bereich von 0,1 bis 5 Gew-%, bezogen auf das Gesamtgewicht des Kosmetikums, vorzugsweise im Bereich von 0,5 bis 2,5 Gew-% liegt.

5. Kosmetikum nach Anspruch 1, **dadurch gekennzeichnet, dass** der Anteil der wässrigen Pflanzenmilch der Frucht von Cocos nucifera im Bereich von 0,1 bis 7 Gew-%, bezogen auf das Gesamtgewicht des Kosmetikums, vorzugsweise im Bereich von 0,2 bis 3,0 Gew-% liegt.

6. Kosmetikum nach Anspruch 1, **dadurch gekennzeichnet, dass** der Anteil des Honigkomplexes im Bereich von 0,1 bis 5 Gew-%, bezogen auf das Gesamtgewicht des Kosmetikums, vorzugsweise im Bereich von 0,1 bis 2,5 Gew-% liegt.

7. Verwendung eines Kosmetikums zur langanhaltenden Feuchthaltung der Haut, wobei das Kosmetikum eine wässrige Pflanzenmilch der Frucht von Elaeis guinensis, eine wässrige Pflanzenmilch der Blätter von Phoenix canariensis, eine wässrige Pflanzenmilch von Reisschalen von Oryza sativa, eine wässrige Pflanzenmilch der Frucht von Cocos nucifera und einen Honigkomplex, bestehend aus Akazienhonig, Eukalyptushonig, Pinienhonig und Lavendelhonig, umfasst.

## Claims

1. A moisture-regulating cosmetic, **characterized in that** it comprises
(a) a watery plant milk of the fruit of Elaeis guinensis,
(b) a watery plant milk of the leaves of Phoenix canariensis,
(c) a watery plant milk of the rice husks of Oryza sativa,
(d) a watery plant milk of the fruit of Cocos nucifera,
(e) a honey complex consisting of acacia honey, eucalyptus honey, pine honey and lavender honey,
and the remainder up to 100 wt % cosmetic auxiliary substances, carrier substances and other active substances, said percentages being based on the total weight of the cosmetic.

2. The cosmetic according to claim 1, **characterized in that** the share of the plant milk of the fruit of Elaeis guinensis is in the range of 0.1 to 10 wt. % based on the total weight of the cosmetic and preferably in the range of 0.5 to 5 wt. %.

3. The cosmetic according to claim 1, **characterized in that** the share of the palm leaf milk of Phoenix canariensis is in the range of 0.1 to 5 wt. % based on the total weight of the cosmetic and preferably in the range of 0.6 to 2 wt. %.

4. The cosmetic according to claim 1, **characterized in that** the share of the rice husk milk of Oryza sativa is in the range of 0.1 to 5 wt. % based on the total weight of the cosmetic and preferably in the range of 0.5 to 2.5 wt. %.

5. The cosmetic according to claim 1, **characterized in that** the share of the watery plant milk of the fruit of Cocos nucifera is in the range of 0.1 to 7 wt. % based on the total weight of the cosmetic and preferably in the range of 0.2 to 3.0 wt. %.

6. The cosmetic according to claim 1, **characterized in that** the share of the honey complex is in the range of 0.1 to 5 wt. % based on the total weight of the cosmetic and preferably in the range of 0.1 to 2.5 wt. %.

7. Use of a cosmetic for the long-lasting moisturizing of the skin, wherein said cosmetic comprises a watery plant milk of the fruit of Elaeis guinensis, a watery plant milk of the leaves of Phoenix canariensis, a watery plant milk of the rice husks of Oryza sativa, a watery plant milk of the fruit of Cocos nucifera and a honey complex consisting of acacia honey, eucalyptus honey, pine honey and lavender honey.

## Revendications

1. Agent cosmétique ayant un effet régulateur d'humidité, **caractérisé en ce qu'**il comprend :
(a) un lait végétal aqueux du fruit d'Elaeis guinensis,
(b) un lait végétal aqueux des feuilles de Phoenix canariensis,
(c) un lait végétal aqueux d'écorces de riz d'Oriza sativa,
(d) un lait végétal aqueux du fruit de Cocos nucifera,
(e) un complexe de miel constitué de miel d'acacia, de miel d'eucalyptus, de miel de pin parasol, et de miel de lavande,
et le reste jusqu'à 100 % en poids d'auxiliaires cosmétiques, de substances de support et d'autres principes actifs, moyennant quoi les données en pourcentage se rapportent au poids total de l'agent cosmétique.

2. Agent cosmétique selon la revendication 1, **caractérisé en ce que** la teneur en lait végétal du fruit d'Elaeis guinensis est dans une gamme de 0,1 à 10 % en poids, rapporté au poids total de l'agent cosmétique, de préférence dans une gamme de 0,5 à 5 % en poids.

3. Agent cosmétique selon la revendication 1, **caractérisé en ce que** la teneur en lait de feuilles de palmier de Phoenix canariensis est dans une gamme de 0,1 à 5 % en poids, rapporté au poids total de l'agent cosmétique, de préférence dans une gamme de 0,6 à 2 % en poids.

4. Agent cosmétique selon la revendication 1, **caractérisé en ce que** la teneur en lait d'écorces de riz d'Oriza sativa est dans une gamme de 0,1 à 5 % en poids, rapporté au poids total de l'agent cosmétique, de préférence dans une gamme de 0,5 à 2,5 % en poids.

5. Agent cosmétique selon la revendication 1, **caractérisé en ce que** la teneur en lait végétal aqueux du fruit de Cocos nucifera est dans une gamme de 0,1 à 7 % en poids, rapporté au poids total de l'agent cosmétique, de préférence dans une gamme de 0,2 à 3,0 % en poids.

6. Agent cosmétique selon la revendication 1, **caractérisé en ce que** la teneur en complexe de miel est dans une gamme de 0,1 à 5 % en poids, rapporté au poids total de l'agent cosmétique, de préférence dans une gamme de 0,1 à 2,5 % en poids.

7. Utilisation d'un agent cosmétique pour maintenir une humidification persistante de la peau, moyennant quoi l'agent cosmétique comprend un lait végétal aqueux du fruit d'Elaeis guinensis, un lait végétal aqueux de feuilles de Phoenix canariensis, un lait végétal aqueux d'écorces de riz d'Oriza sativa, un lait végétal aqueux du fruit de Cocos nucifera et un complexe de miel constitué de miel d'acacia, de miel d'eucalyptus, de miel de pin parasol, et de miel de lavande.
